# EUROPEAN PATENT APPLICATION

(11) **EP 1 420 592 A1**
(43) Date of publication of application: **19.05.2004**
(21) Application number: 03257141.6
(22) Date of filing: 12.11.2003
(51) Int. Cl.: H04N 7/18, A61B 1/267, A61B 5/11, G01H 9/00

(54) **Vibrating object observing system**

(30) Priority: 18.11.2002 JP 2002333086
(71) Applicant: MACHIDA ENDOSCOPE CO., LTD, Bunkyo-ku, Tokyo (JP)
(72) Inventor: Miyagi, Kunihiko c/o Machida Endoscope Co., Ltd., Bunkyo-ku Tokyo (JP); Misawa, Masayuki c/o Machida Endoscope Co., Ltd., Bunkyo-ku Tokyo (JP)
(74) Representative: Hallybone, Huw George

(57) **Abstract**

An observing system(S) for observing a vocal cord(B), as a vibrating object, of a person to be inspected(A) comprises an image-pickup section(1) for picking up an image of the vocal cord(B) at a constant cycle, an extracting section(21a) for extracting a basic frequency of a generated voice, a frequency dividing ratio setting section(26) for variably setting a frequency dividing ratio with respect to the extracted basic frequency, a frequency dividing section(21b) for dividing the basic frequency at the set frequency dividing ratio, a trigger output section(21c) for outputting a trigger signal at the divided frequency, and video image making sections(21d, 22) capable of outputting only an image picked up by the image pickup section(1) immediately after the output of each trigger signal.

Owing to the above arrangement, a video image, which looks as if a vibrating object were moving in a slow motion manner, can be obtained.

## Description

### BACKGROUND OF THE INVENTION

This invention relates to an apparatus for observing a vibrating object such as, for example, a vocal cord.

As a system for observing the vocal cord of a person to be inspected, there is known a system comprising a combination of an endoscope and a larynx stroboscopy which is used as a light source of the endoscope (see, for example, JOHNS (p 797, vol. 12, 6th number of 1996) issued by Tokyoigakusha. The endoscope is inserted into the larynx through the mouth. The larynx stroboscopy is used for extracting a basic frequency of the voice of a person to be inspected and emitting a stroboscopic light while shifting the phase little by little with the same frequency as the extracted frequency. By doing so, an image, which looks as if the vocal cord were moving in a slow motion manner, can be observed through the endoscope.

Since it is necessary for the above larynx stroboscopy to illuminate the larynx instantaneously brightly and yet intermittently at an extremely short cycle, a stroboscope light source device which is high in luminance and high in performance is required. This makes the larynx stroboscopy expensive.

The present invention has been accomplished in view of the above situation. It is, therefore, an object of the present invention to provide an inexpensive system, in which a vibrating object such as a vocal cord can be observed with a simple structure and without using a light source which is high in luminance and high in performance.

### SUMMARY OF THE INVENTION

In order to solve the above problems, the present invention provides a system for projecting a vibrating object onto an image projecting means for observation, which system is characterized by comprising an image-pickup section for picking up an image of the object at a constant cycle, a frequency detecting section for detecting the frequency of the vibration, a frequency dividing ratio setting section for variably setting a frequency dividing ratio with respect to the detected frequency, a trigger output section for outputting a trigger signal at a frequency obtained by dividing the detected frequency at the frequency dividing ratio set by the frequency dividing ratio setting section, and a video image making section capable of outputting to the image projecting means only an image picked up by the image pickup section immediately after each trigger signal is outputted.

According to the above characterized construction, an image can be obtained which is suitable for observing a vibrating state of an object in which the vibrating object looks as if it were virtually moving in a slow motion manner. Owing to this characterized construction, it is no more required to employ a light source which is high in luminance and high in performance and the structure can be simplified. Moreover, the system can be made inexpensive.

The frequency dividing ratio setting section may have a handle for manually adjusting the frequency dividing ratio within a predetermined range, or it may have a frequency dividing ratio automatic setting function for automatically setting a frequency dividing ratio suitable for observing a vibrating state of the vibrating object. According to the first mentioned manually setting system, the structure can be more simplified and the system can be made more inexpensive. According to the second mentioned automatically setting system, the vibration observing operation can be conducted in a very simple and easy manner. This automatic setting operation may be conducted based on feedback from the video image making section, or it may be conducted based on the detected frequency. The automatic setting operation may also be conducted based on the image pickup data obtained by picking up an image of the object at a constant cycle.

It is preferable that the video image making section includes an image storage section for receiving for storage therein an image for one field from the image pickup section so as to output the image to the image projecting means, and an image storage control section for controlling the storing operation of the image storage section in accordance with the trigger signal. Owing to this arrangement, a new image is overwritten on the image storage control section every time the trigger signal is outputted and the newly overwritten image can be projected until receipt of next trigger signal. It is good enough for the image storage section to have a storage capacity for one field.

It is accepted that the image pickup section includes an endoscope which can be inserted into a larynx of a person to be inspected so that an image of a vocal cord of the person can be obtained; and the frequency detecting section includes a voice collecting section for collecting a voice generated by the person, and an extracting section for extracting a basic frequency of the collected voice as the vibrating frequency to be detected. Thereby, the vibrating object observing system is provided as a vocal cord observing system in which a vocal cord serves as an object to be observed. The light source of the endoscope is not required to be high in luminance. A light source having a standard luminance is good enough. Thus, the system can be made inexpensive positively. The endoscope itself may be provided with a photoelectric conversion section such as a solid image pickup device for converting an optical image into an electric signal. It may also be accepted that a photoelectric conversion section is connected to the endoscope as a separate component. The photoelectric conversion section is connected with an electric/video image conversion section such as a camera control unit for converting an electric signal into a video image signal.

As a processing apparatus used for this vocal cord observing system, the apparatus preferably comprise a housing in which the extracting section, the frequency dividing ratio setting section, the trigger output section, the video image making section, a connecting terminal connected directly or indirectly to the endoscope, a connecting terminal connected to the image projecting means, and a connecting terminal connected to the voice collecting section are mounted. Owing to this arrangement, the system construction can be made compact. In addition, a general endoscope apparatus, a television monitor and a microphone can be used as the image pickup section, the image projecting means and the collecting section respectively. Merely by connecting them to the vocal cord observing processing apparatus, a vocal cord observing system can easily be constructed.

It is preferable that the extracting section, the frequency dividing ratio setting section, the trigger output section and the video image making section are received in the housing. The various connecting terminals are preferably disposed at, for example, the external surface of the housing so that it can easily be accessed from the outside. The handle of the frequency dividing ratio setting section in the manually setting system is disposed at, for example, the outer surface of the housing as in the case with the various connecting terminals.

The electric/video image converting section of the image pickup section may be constructed separately from the vocal cord observing processing apparatus. It may also be received in the housing of the vocal cord observing processing apparatus. In case the electric/video image converting section is separately constructed, the housing of the processing apparatus is provided with the connecting terminal which is directly connected to the electric/video image converting section and indirectly to the endoscope. In case the electric/video image converting section is received in the housing, the housing is provided with an input terminal of the electric/video image converting section which serves as a connecting terminal connected to the endoscope.

### BRIEF DESCRIPTION OF THE DRAWINGS

FIG. 1 is a schematic block diagram showing a vocal cord observing system according to one embodiment of the present invention.
FIG. 2 is a chart showing one example (frequency dividing ratio: 5) of the processing conducted by the vocal cord observing system.
FIG. 3 is a chart showing another example (frequency dividing ratio: 3) of the processing conducted by the vocal cord observing system.

### DETAILED DESCRIPTION OF THE INVENTION

One embodiment of the present invention will be described hereinafter with reference to the drawings.

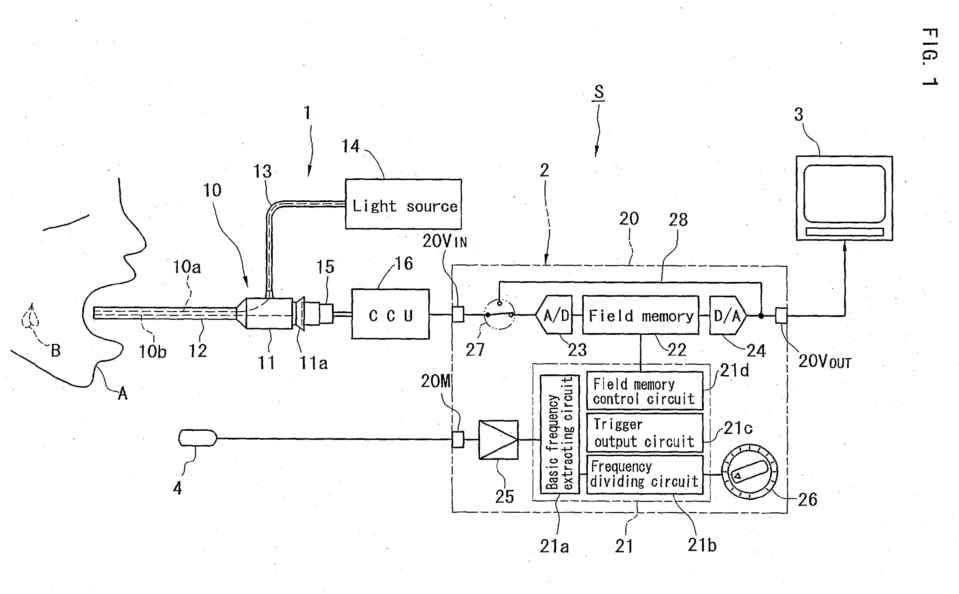

FIG. 1 shows a vocal cord observing system S for observing the vibrating manner of the vocal cord B of a person A to be inspected. The vocal cord B serves as an object to be observed.

The vocal cord observing system S comprises an image pickup section 1, a vocal cord observing processing apparatus 2 and a television monitor 3. The vocal cord observing system S serves as a vibrating object observing system. The television monitor 3 serves as an image projecting means.

The image pickup section 1 includes an endoscope 10, a camera head unit 15 and a camera control unit 16.

The camera head unit 15 serves as an optoelectronic converting section. The camera control unit 16 serves as an electric/video image converting section.

As known, the endoscope 10 includes a main body section 11 and an insertion section 12 extending from the main body section 11. The insertion section 12 is designed in such a manner as to be insertable into the larynx of the person A to be inspected. A light guide 10a and an image guide 10b are received in the main body section 11 and the insertion section 12. The light guide 10a and the image guide 10b are each formed of a bundle of optical fibers.

A basal end part of the light guide 10a is optically connected to a light source 14 through a light cable 13, and a distal end part thereof reaches a distal end face of the insertion section 12. Owing to this arrangement, an illumination light emitted from the light source 14 is allowed to pass through the light guide 10a and outputted from the distal end face of the insertion section 12.

A distal end part of the image guide 10b is faced with the distal end face of the insertion section 12 and a basal end part thereof is optically connected to an ocular part 11a of a basal end part of the main body section 11. Owing to this arrangement, an optical image made incident to the distal end face of the image guide 10b is allowed to pass through the image guide 10b and transferred to the ocular part 11a.

The camera head unit 15 is optically connected to the ocular part 11a of the endoscope 10. The camera head unit 15 is provided with a solid image pickup device for optoelectronically converting an optical image coming from the ocular part 11a. The camera head unit 15 is connected with the camera control unit 16. When receiving the electric signal from the camera head unit 15, the camera control unit 16 makes video image data for one field at an interval of 1/60 sec, for example, according to the NTSC system.

The endoscope 10 may be a hard endoscope. Or the endoscope 10 may be an electronic endoscope with a solid image pickup device built therein instead of the image guide 10b. The camera unit 15 and the camera control unit 16 may be integrally mounted on the endoscope 10.

The vocal cord observing processing apparatus 2 will now be described.

The vocal cord observing processing apparatus 2 includes a control module 21, a field memory 22 as an image storage section and a housing 20 for receiving therein the control module 21 and the field memory 22.

The field memory 22 has a memory capacity just enough for one field image data. The field memory 22 is connected to a video input terminal 20V_{IN} through an A/D converter 23. The video input terminal 20 V_{IN} is disposed at an outer surface of the housing 20. The camera control unit 16 is removably connected to the video input terminal 20V_{IN}. The video input terminal 20V_{IN} serves as a connecting terminal connected to the image pickup section and therefore to the endoscope.

The image data coming from the camera control unit 16 are digitally converted by the A/D converter 23 and stored in the field memory 22. The storage data in the field memory 22 are overwritten with new data every time image data coming from the camera control unit 16 are inputted therein.

The field memory 22 is connected to a video output terminal 20V_{OUT} through a D/A converter. The video ouput terminal 20 V_{OUT} is disposed at the outer surface of the housing 20. The TV monitor 3 is removably connected to the video output terminal 20V_{OUT}. The video ouput terminal 20 V_{OUT} serves as a connecting terminal connected to the image projecting means.

The digital image data stored in the field memory 22 are called at an interval of 1/60 sec in accordance with NTSC system, converted back to a video image signal by the D/A converter 24 and then sent to the television monitor 3 so as to be shown.

The control module 21 is provided with a basic frequency extracting circuit 21a, a frequency dividing circuit 21b, a trigger output circuit 21c and a field memory control circuit 21d.

The basic frequency extracting circuit 21a serves as an extracting section.

The trigger output circuit 21c serves as a trigger output section.

The field memory control circuit 21d serves as an image storage control section

The basic frequency extracting circuit 21a is connected to a microphone input terminal 20 M disposed at an outer surface of the housing 20 through a microphone amplifier 25. A microphone 4 as a voice collecting section is removably connected to the microphone input terminal 20M.

The microphone input terminal 20M serves as a connecting terminal connected to the voice collecting section.

The voice collected by the microphone 4 is amplified by the microphone amplifier 25 and then inputted into the extracting circuit 21a. The extracting circuit 21a extracts the basic frequency of the inputted voice. This basic frequency is coincident with the vibration frequency of the vocal cord B of the object to be observed.

A "frequency detecting section" is constituted by the microphone 4 and the basic frequency detecting circuit 21a.

The frequency dividing circuit 21b is connected to the basic frequency detecting circuit 21a, and this frequency dividing circuit 21b is connected with a dial-shaped handle 26 disposed at the outer surface of the housing 20. By turning this handle 26, the frequency dividing ratio with respect to the basic frequency can be set within a range of, for example, 2 to 16. Of course, the range for setting is not limited to this. The range for setting may be larger or smaller than the above mentioned range.

In the frequency dividing circuit 21b, the frequency extracted by the extracting circuit 21a is divided at the frequency dividing ratio which is set by the handle 26.

A "frequency dividing ratio setting section" is constituted by the frequency dividing circuit 21b and the handle 26.

The trigger output circuit 21c is connected to the frequency dividing circuit 21b. The trigger output circuit 21c outputs pulse-like trigger signal having the same frequency as the divided frequency obtained by the frequency dividing circuit 21b to the field memory control circuit 21d.

The field memory control circuit 21d is connected to the field memory 22. The field memory control circuit 21d conducts such control operation with respect to the field memory 22 as to make the memory 22 into a overwritable state every time it receives the trigger signal and to make the memory 22 into a non-overwritable state after the memory 22 is overwritten till the next trigger signal is received.

A "video image making section" is constituted by the field memory control circuit 21d and the field memory 22.

A mode changeover switch 27 is manually controllably mounted on the housing 20 of the vocal cord observing processing apparatus 2. The mode changeover switch 27 is circuit-wise interposed between the video input terminal 20V_{IN} and the A/D converter 23.

A bypass road 28 extends from this mode changeover switch 27. The bypass road 28 is allowed to bypass the A/D converter 23, the field memory 22 and the D/A converter 24 and connected to the wiring between the D/A converter 24 and the video output terminal 20V_{OUT}.

The mode changeover switch 27 can manually be switched between a processing mode position and a non-processing mode position. In the processing mode position, the switch 27 interconnects the video input terminal 20V_{IN} and the A/D converter 23. In the non-processing mode position, it connects the video input terminal 20V_{IN} to the bypass road 28. Owing to this arrangement, one of the processing mode and the non-processing mode can be selected.

In the non-processing mode, the video image data obtained by the image pickup section 1 are bypassed through the bypass road 28 so that it is directly sent to the television monitor 3 without being subjected to processing in the vocal cord observing apparatus 2. That is, the image observed through the endoscope 10 is directly shown in the television monitor 3. On the other hand, in the processing mode, the video image observed through the endoscope is subjected to processing in the vocal cord observing processing apparatus 2 and then shown in the television monitor 3.

The method of use and operation of the vocal cord observing system S thus constructed will now be described.

First, the light source 14 of the endoscope 10 is turned on, the insertion section 12 is pushed into the buccal cavity of the person A and an image of the voice cord B is picked up. At that time point, the mode changeover switch 27 is good to be in the non-processing mode so that the inserting operation of the endoscope 10 can be conducted in the normal manner.

The microphone 4 is arranged in a position where the voice of the person A can be collected. After the mode changeover switch 27 is switched into the processing mode, the person A is asked to pronounce the sound of, for example, "Uh........ " continuously.

This voice of the person A is collected into the microphone 4 and delivered to the extracting circuit 21a via the microphone amplifier 25. By this, the extracting circuit 21a extracts the basic frequency of the voice of the person A.

Then, the frequency dividing circuit 21b divides the extracted basic frequency at the frequency dividing ratio set by the handle 26 so as to compute a dividing frequency. And the trigger output circuit 21c outputs a trigger signal with the dividing frequency.

In response to this trigger signal, the field memory control circuit 21d overwrites video image data for one field, which video image was picked up by the camera control unit 16 immediately after each trigger signal, on the field memory 22. By this, the image shown in the television monitor 3 is switched to a rewritten new image.

At that time, by adjusting the setting frequency dividing ratio with the handle 26, the movement of the vocal cord B can be shown as if the vocal cord B were moving in a slow motion manner.

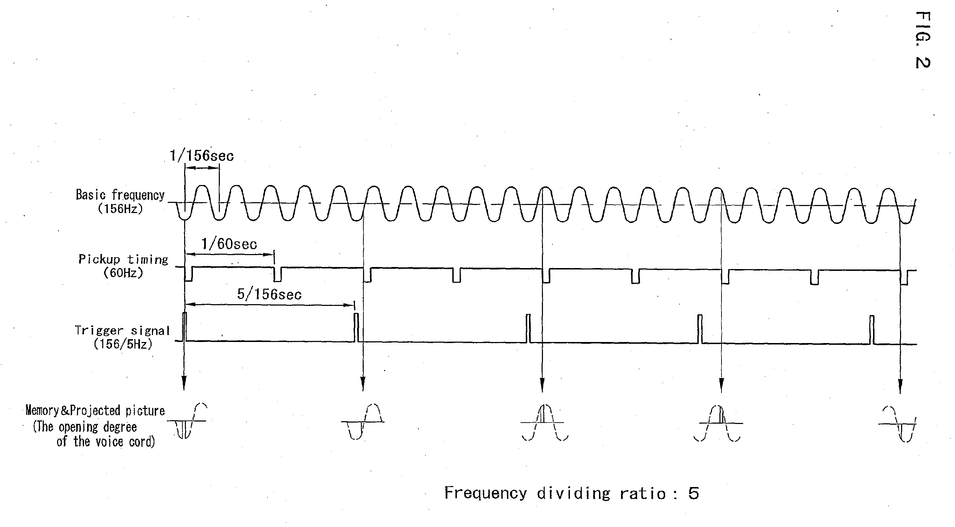

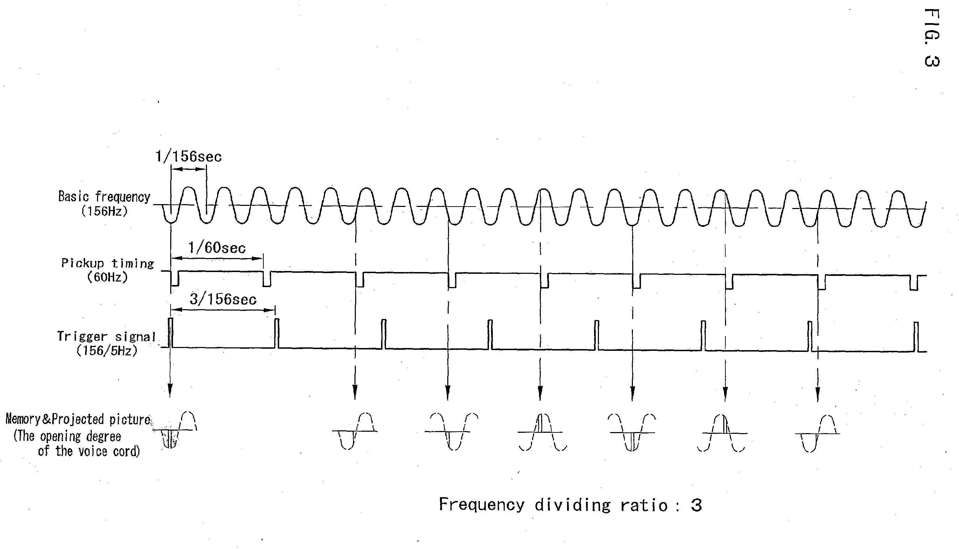

The processing conducted by the vocal cord observing processing apparatus 2 will now be described specifically with reference to the charts of FIGS. 2 and 3.

Let's presume here that the basic frequency of the voice generated by the person A is, for example, 156 Hz as shown in FIG. 2. Irrespective of this basic frequency (156 Hz ), the camera head unit 15 picks up the image of the vocal cord B at the timing of a constant frequency 60 Hz according to the NTSC system. Even if the data of the image picked up at the above-mentioned 60 Hz are directly outputted, it is normally impossible to obtain such a video image that the vocal cord B is regularly opened and closed.

Again, let's us presume here that the frequency dividing ratio is set to, for example, 5 by the handle 26. The trigger output circuit 21c outputs a trigger signal with a frequency (156/5 Hz here) obtained by dividing the basic frequency with the set frequency dividing ratio (5 here). The field memory control circuit 21d stores the image data immediately after the output of the trigger signal in the field memory 22, and the circuit 21 keeps the outputting to the monitor 3 until the image data are rewritten in accordance with the next trigger signal. By this, a video image of the vocal cord B which is regularly changed every trigger signal (every 5/156 sec. here) is shown in the television monitor 3.

That is, even if the original image data are irregular and discontinuous, by selecting a suitable extracting interval and outputting the image data at the interval to the TV monitor, there can be obtained a video image of the vibration of the vocal cord B which is regularly sequentially changed as if virtually moving in a slow motion manner, and the video image can be observed in detail.

By turning the handle 26, a frequency dividing ratio, which is matched with the basic frequency of the generated voice, can be set and a virtual slow motion video image suitable for observation can be obtained as shown in FIG. 2.

On the other hand, presuming that the frequency dividing ratio of the handle 26 is set to "3" as shown in FIG. 3, the image shown in the television monitor 3 becomes such that the vocal cord B is irregularly changed. Thus, such an image is not suitable for observation.

As discussed, in the vocal cord observing system S, vibration of the vocal cord can be observed with a simple structure. The light source 14 for illumination is not required for having a special function. It is good enough only if it has a function enough to illuminate the vocal cord. Therefore, a general endoscope can be used and the cost can be reduced extensively compared with the conventional system which requires a stroboscope for intermittently emitting light high in luminance at a short cycle.

The present invention is, by no means, limited to the above embodiment. Instead, many changes and modifications can be made.

For example, the frequency dividing ratio may be automatically set by an apparatus 20 instead of the manual operation through the handle 26. That is, the frequency dividing ratio setting section may have such a frequency dividing ratio automatic setting function for automatically setting a frequency dividing ratio which is suitable for observing a vibrating state in which the vocal cord B is virtually moving in a slow motion manner. For automatically setting a frequency dividing ratio, the frequency dividing ratio automatic setting section may apply an image processing upon receipt of feedback of a video image signal which is to be outputted into the television monitor. Or the automatic setting section may apply an image processing upon receipt of image signal for each 60 Hz by the camera control unit 16. Or the automatic setting section may apply an arithmetic computation processing based on the basic frequency of the voice extracted by the extracting circuit 21a and the image pickup frequency (60 Hz) under the NTSC system.

It is accepted that the camera control unit 16 is received in the housing 20 of the vocal cord observing processing apparatus 2 and a signal input terminal of the camera control unit 16 is disposed at the outer surface of the housing 20. The signal input terminal serves as a connecting terminal connected to the endoscope.

The present invention is not limited to be used for observing the vibration of the vocal cord but it may also be widely applied for observing a vibrating object such as, for example, an industrial product.

## Claims

1. A system(S) which projects a vibrating object(B) onto an image projecting means(3) for observation,
**CHARACTERIZED in that** said system(S) comprising:
an image-pickup section(1) for picking up an image of said object at a constant cycle;
a frequency detecting section(4,21a) for detecting the frequency of said vibration;
a frequency dividing ratio setting section(21b,26) for variably setting a frequency dividing ratio with respect to the detected frequency;
a trigger output section(21c) for outputting a trigger signal at a frequency obtained by dividing the detected frequency at the frequency dividing ratio set by said frequency dividing ratio setting section(21b,26); and
a video image making section(21d,22) capable of outputting to said image projecting means(3) only an image picked up by said image pickup section immediately after each trigger signal is outputted.

2. A vibrating object observing system(S) according to claim 1, wherein said frequency dividing ratio setting section(21b,26) has a handle(26) for manually adjusting said frequency dividing ratio within a predetermined range.

3. A vibrating object observing system(S) according to claim 1, wherein said frequency dividing ratio setting section has a frequency dividing ratio automatic setting function for automatically setting a frequency dividing ratio suitable for observing a vibrating state of said vibrating object(B).

4. A vibrating object observing system(S) according to claim 1, wherein said video image making section(21d,22) includes:
an image storage section(22) for receiving for storage therein an image for one field from said image pickup section(1) so as to output the image to said image projecting means(3); and
an image storage control section(21d) for controlling the storing operation of said image storage section(22) in accordance with on said trigger signal.

5. A vibrating object observing system(S) according to claim 1, wherein said image pickup section(1) includes an endoscope(10) which can be inserted into a larynx of a person(A) to be inspected so that an image of a vocal cord(B) of said person(A) can be obtained;
said frequency detecting section(4,21a) includes a voice collecting section(4) for collecting a voice generated by said person(A), and an extracting section(21a) for extracting a basic frequency of the collected voice as said vibrating frequency to be detected;
thereby said vibrating object observing system(S) is provided as a vocal cord observing system in which a vocal cord(B) serves as an object to be observed.

6. A processing apparatus(2) used for said vocal cord observing system(S) according to claim 5, said apparatus(2) comprising a housing(20) in which said extracting section(21a), said trigger output section(21c), said video image making section(21d,22), a connecting terminal(20V_{IN}) connected directly or indirectly to said endoscope(10), a connecting terminal(20V_{OUT}) connected to said image projecting means(3), and a connecting terminal(20M) connected to said voice collecting section(4) are mounted.
